# EUROPEAN PATENT APPLICATION

(11) **EP 2 468 313 A1**
(43) Date of publication of application: **27.06.2012**
(21) Application number: 11189603.1
(22) Date of filing: 17.11.2011
(51) Int. Cl.: A61L 27/04, A61L 27/30, A61F 2/01, C23C 16/40

(54) **Ceramic coated orthopaedic implants**

(30) Priority: 19.11.2010 US 950073
(71) Applicant: DePuy Products, Inc., Warsaw, IN 46581 (US)
(72) Inventor: Langhorn, Jason B, Warsaw, IN Indiana 46581 (US); Overholser, Ronald W, Warsaw, IN Indiana 46581 (US); Smith, Bryan J, Warsaw, IN Indiana 46581 (US)
(74) Representative: Belcher, Simon James

(57) **Abstract**

A method of making an orthopaedic implant component uses a metal component which has been pressed using hot isostatic pressing and homogenised. A ceramic coating is provided on the pressed and homogenised component by depositing a first band of the ceramic coating upon the pressed and homogenised metal substrate, and depositing a second band of the ceramic coating upon the first band of the ceramic coating.

## Description

The present invention relates to coatings for metal substrates, such as those used to prepare medical implants, and to methods of making such implants. Such coatings can enhance resistance to wear, scratching and corrosion.

Once installed, metallic orthopaedic implants are vulnerable to deterioration caused by scratching, wear, or otherwise damaging or corrosive processes that can occur *in situ.* Damaged implants may exhibit diminished performance, and in some cases must be repaired or replaced, and the complex and often physically traumatic surgical procedures necessary for doing so can delay the patient's progress towards rehabilitation. Furthermore, longer-lasting orthopaedic implants are of increasing interest due to demographic trends such as the increased life expectancy of implant recipients and the need for orthopaedic intervention among younger subjects (for example, due to sports injury, excessive body weight leading to joint stress, or poor health maintenance).

Implants comprising metallic substrates, including such materials as steel, cobalt, titanium, and alloys thereof, are also vulnerable to damage or mechanically-assisted corrosion that can lead to loss of structural integrity, scratching or abrasive wear, increased wear rates and reduction of implant performance.

Traditional approaches for improving resistance to wear and scratching of metallic orthopaedic implants have included surface treatments such as ion implantation, gas nitriding, high temperature oxidation, and coating techniques (for example as disclosed in US-A-2007/0078521). However, certain limitations such as inability to provide an optimal level of peak hardness, poor adherence of coatings to underlying substrates, and economic feasibility may abridge the utility of some of these traditional methods.

The present invention relates to the discovery that the scratch, corrosion and wear resistance and adhesion of a ceramic coating formed on metallic orthopaedic implant components may be improved by controlling the process parameters used to prepare the metal substrate prior to coating the substrate. The present invention also relates to the discovery that the scratch, corrosion and wear resistance and adhesion of a ceramic coating formed on metallic orthopaedic implant components may be improved by using a coating comprising multiple "thin" layers of ceramic instead of fewer thicker layers. In addition, the present invention also relates to the discovery that the optimal composition of the outer articular surface of a ceramic-coated orthopaedic implant component may advantageously be varied with the material used for the bearing that bears against the outer articular surface.

The invention makes use of materials which have been subjected to a pressing step using hot isostatic pressing techniques. When the pressing step is referred to in this document, it can be performed using hot isostatic pressing.

Accordingly, the invention provides a method of making an orthopaedic implant component comprising the steps of obtaining a metal orthopaedic implant component that has been pressed using a hot isostatic pressing technique and homogenised, and depositing a ceramic coating on the pressed and homogenised component by depositing a first band of the ceramic coating upon the pressed and homogenised metal substrate and depositing a second band of the ceramic coating upon said first band of the ceramic coating.

The step of obtaining a metal orthopaedic implant component that has been pressed using a hot isostatic pressing technique and homogenised can include obtaining a metal orthopaedic implant component with a surface that has been pressed, homogenised and from which 0.5 to 1 mm of pressed and homogenised metal has been removed from at least a portion of the metal orthopaedic implant component. In a more particular embodiment, the pressed and homogenised metal is removed through at least one of the following material removal processes: grinding; machining; and polishing.

The step of depositing a first band of the ceramic coating may comprise depositing (for example using a chemical vapour deposition (CVD) technique) a layer of titanium nitride, titanium carbonitride or both titanium nitride and titanium carbonitride.

The step of depositing a second band may comprise depositing at least one layer of titanium nitride, titanium carbonitride or both titanium nitride and titanium carbonitride.

The method may include a step of depositing an outer band of the ceramic coating upon the second band, with the outer band defining the outer articular surface of the orthopaedic implant component. In one particular embodiment, the outer band comprises alumina; an additional bonding band may be deposited between the second band and the alumina of the outer band. Alternatively, the outer band may comprise a layer of titanium nitride, titanium carbonitride or both titanium nitride and titanium carbonitride.

The step of depositing a second band may comprise depositing (for example using a chemical vapour deposition technique) a plurality of layers, each layer comprising titanium nitride, titanium carbonitride or both titanium nitride and titanium carbonitride. In this embodiment, the thickness of the layer of the first band may be greater than the thickness of each layer in the second band. The number of layers which might be deposited in the second band can be at least 2, or at least 5, or at least 30. The number of layers which might be deposited in the second band might be not more than 100, or not more than 50. Each layer can comprise titanium nitride, titanium carbonitride or both titanium nitride and titanium carbonitride.

Optionally, the second band comprises alumina and in which the alumina defines the outer articular surface of the orthopaedic implant component.

Optionally, the method includes depositing a bonding band between the first band and the alumina of the second band.

Optionally, the step of depositing a second band comprises depositing a plurality of layers, for example at least 2 layers or at least 5 layers or at least 30 layers, for example not more than about 100 layers, or not more than 50 layers. Each layer can comprise titanium nitride, titanium carbonitride or both titanium nitride and titanium carbonitride. The layers can be deposited using a chemical vapour deposition (CVD) technique.

Optionally, the method includes depositing a third band of the ceramic coating upon the second band of the ceramic coating.

Optionally, the third band comprises alumina, and the alumina defines the outer articular surface of the orthopaedic implant component. Preferably the third band is deposited using a chemical vapour deposition technique.

Optionally, the method includes depositing a bonding band between the second band and the alumina of the third band.

Optionally, the first band comprises a plurality of layers of titanium nitride, titanium carbonitride or both titanium nitride and titanium carbonitride. The layers can be deposited using a chemical vapour deposition technique.

Optionally, the second band comprises alumina and in which the alumina defines the outer articular surface of the orthopaedic implant component.

Optionally, the method includes depositing a bonding band between the first band and the alumina of the second band.

Optionally, the second band comprises a plurality of layers, each layer comprising titanium nitride, titanium carbonitride or both titanium nitride and titanium carbonitride.

The number of layers which might be deposited in the second band can be at least 2, or at least 5, or at least 30. The number of layers which might be deposited in the second band might be not more than 100, or not more than 50. The number of layers which might be deposited in the second band can be at least 2, or at least 5, or at least 30. The number of layers which might be deposited in the second band might be not more than 100, or not more than 50. The layers can be deposited using chemical vapour deposition techniques.

Optionally, the method includes depositing a third band of the ceramic coating upon the second band of the ceramic coating.

Optionally, the third band comprises alumina and the alumina defines the outer articular surface of the orthopaedic implant component. Preferably the third band is deposited using a chemical vapour deposition technique.

Optionally, the method includes depositing a bonding band between the second band and the alumina of the third band.

The invention also provides an orthopaedic implant kit comprising a first component having an outer articular surface and a second component having an outer bearing surface sized and shaped to articulate against the articular surface of the first component. The first orthopaedic implant component includes a metal substrate surface that is substantially free from interdendritic carbides and a ceramic coating on the metal substrate. The ceramic coating defines the outer articular surface of the first component. The ceramic coating has a total thickness of about 3 µm to 20 µm and includes a material selected from the group consisting of titanium carbide, titanium nitride, titanium carbonitride, and both titanium nitride and titanium carbonitride.

The outer bearing surface of the second component can be defined by a material selected from the group consisting of metal and ceramic and the ceramic coating of the first component has an outer surface comprising a material selected from the group consisting of titanium carbide, titanium nitride, titanium carbonitride, and both titanium nitride and titanium carbonitride.

The ceramic coating can include a first band and a second band. The first band comprises titanium nitride, titanium carbonitride, or both titanium nitride and titanium carbonitride covering the substrate surface. The second band comprises a plurality of layers of titanium nitride, titanium carbonitride, or both titanium nitride and titanium carbonitride covering the first band. The first band has a thickness greater than the thickness of each layer in the second band.

The ceramic coating may include a third band. The third band may have a thickness greater than the thickness of each layer in the second band. In one more particular embodiment the third band comprises titanium nitride, titanium carbonitride, or both titanium nitride and titanium carbonitride covering the second band, with the third band having a thickness greater than the thickness of each layer in the second band. More particularly, the third band may comprise a single layer having a thickness of from about 2 to 15 µm.

The ceramic coating can include a third band comprising alumina which covers the second band. The third band can have a thickness greater than the thickness of each layer in the second band. The third band may comprise a single layer having a thickness of from about 2 to 15 µm.

The second band can comprise about 2 to 50 layers of ceramic, each layer comprising titanium nitride, titanium carbonitride, or both titanium nitride and titanium carbonitride. The second band may comprise about 5 to 50 layers of ceramic, each layer comprising titanium nitride, titanium carbonitride, or both titanium nitride and titanium carbonitride. The second band may comprise about 30 to 50 layers of ceramic, each layer comprising titanium nitride, titanium carbonitride, or both titanium nitride and titanium carbonitride.

The second band can comprise a plurality of layers of ceramic, each layer having a thickness less than about 0.5 µm, for example less than about 0.2 µm.

The first band can have a thickness of about 2 to 3 µm, for example about 2.5 µm.

The ceramic coating can have a total thickness of 10 to 15 µm, for example 14 to 15 µm. The first band can comprise a single layer of ceramic comprising titanium nitride, titanium carbonitride, or both titanium nitride and titanium carbonitride, the single layer having a thickness of about 2 to 3 µm. The second band can comprise about 30 to 50 layers of ceramic, each layer comprising titanium nitride, titanium carbonitride, or both titanium nitride and titanium carbonitride, each layer having a thickness less than about 0.2 µm. A third band comprising alumina can cover the second band, the third band having a thickness of about 2 to 10 µm.

The invention also provides an orthopaedic implant component having an outer articular surface. The orthopaedic implant component comprises a metal substrate surface and a ceramic coating on the metal substrate surface defining the outer articular surface of the implant component. The ceramic coating includes a first band and a second band. The first band comprises titanium nitride, titanium carbonitride, or both titanium nitride and titanium carbonitride covering said substrate surface. The second band comprises a plurality of layers of titanium nitride, titanium carbonitride, or both titanium nitride and titanium carbonitride covering said first band. The ceramic coating includes a portion that exhibits no acoustic emission peaks characteristic of Lc2 chipping or buckling spallation type cracking events per millimetre of scratch length from a 200 µm radius diamond indenter under a 20 N constant load. Chipping and buckling spallation Lc2 events, together with acoustic emission characteristics are defined in ASTM C1624-05.

The ceramic coating can include a portion that has fewer than five acoustic emission peaks characteristic of Lc2 chipping or buckling spallation per millimetre of scratch length from a 200 µm radius diamond indenter under a 40 N constant load as measured according to ASTM C1624-05.

The ceramic coating can include a portion that has fewer than two acoustic emission peaks characteristic of Lc2 chipping or buckling spallation per millimetre of scratch length from a 200 µm radius diamond indenter under a 40 N constant load as measured according to ASTM C1624-05.

The ceramic coating can include a portion that has no acoustic emission peaks characteristic of Lc2 chipping or buckling spallation per millimetre of scratch length from a 200 µm radius diamond indenter under a 25N constant load as measured according to ASTM C 1624-05.

The ceramic coating can include a portion that has no acoustic emission peaks characteristic of Lc2 chipping or buckling spallation per millimetre of scratch length from a 200 µm radius diamond indenter under a 28 N constant load as measured according to ASTM C 1624-05.

The ceramic coating can include a portion that has no acoustic emission peaks characteristic of Lc2 chipping or buckling spallation per millimetre of scratch length from a 200 µm radius diamond indenter under a 30 N constant load as measured according to ASTM C 1624-05.

The ceramic coating may also comprise an outer band covering the second band, the outer band defining an articulating surface of the implant component. The outer band may comprise alumina or alternatively may comprise titanium carbide, titanium nitride, titanium carbonitride or both titanium nitride and titanium carbonitride.

The inner band may include a layer of titanium nitride, titanium carbonitride or both titanium nitride and titanium carbonitride. The thickness of the layer of the inner or first band may be greater than the thickness of each layer of the second band. In embodiments with an outer band, the thickness of the layer of the outer band may be greater than the thickness of each layer of the second band.

The ceramic coating may have a thickness of 10 to 20 µm.

The layer of the first band may have a thickness of about 2 to 3 µm, the layer of the outer band may have a thickness of about 5 µm, and the second band may have a thickness of about 4 to 14 µm. It can be preferred that the second band has a thickness of about 5 µm.

Embodiments of the invention are described below by way of example with reference to the accompanying drawings, in which:
FIG. 1 shows, diagrammatically, a cross-section of a scratch-, wear-, and corrosion-resistant articular surface of an orthopaedic implant component;
FIG. 2 shows the components of an example of a knee implant system in which a coating is applied to the articular surfaces of the femoral component;
FIG. 3 shows the components of an example of a hip implant system in which a coating is applied to the articular surfaces of the femoral head component;
FIG. 4 shows transmission electron microscope (TEM) images of a conventional "dual layer" TiN/TiCN/alumina coating;
FIG. 5 shows transmission electron microscope (TEM) images of a multilayer coating that was prepared in accordance with the present invention;
FIGS. 6A-6E provide magnified images of surfaces that were respectively coated with coatings according to the invention and conventional coatings and subjected to scratch testing in order to compare the mechanical performance of the respective coatings;
FIG. 7 provides magnified images from an SEM analysis of polished cross sections of (A) conventional coatings and (B) coatings according to the invention through 40 N constant load scratches, perpendicular to the scratch direction;
FIGS. 8A and 8B provide magnified images from a metallographic analysis of polished cross-sections of cast Co-28Cr-6Mo which have been pressed using a hot isostatic pressing technique and homogenised;

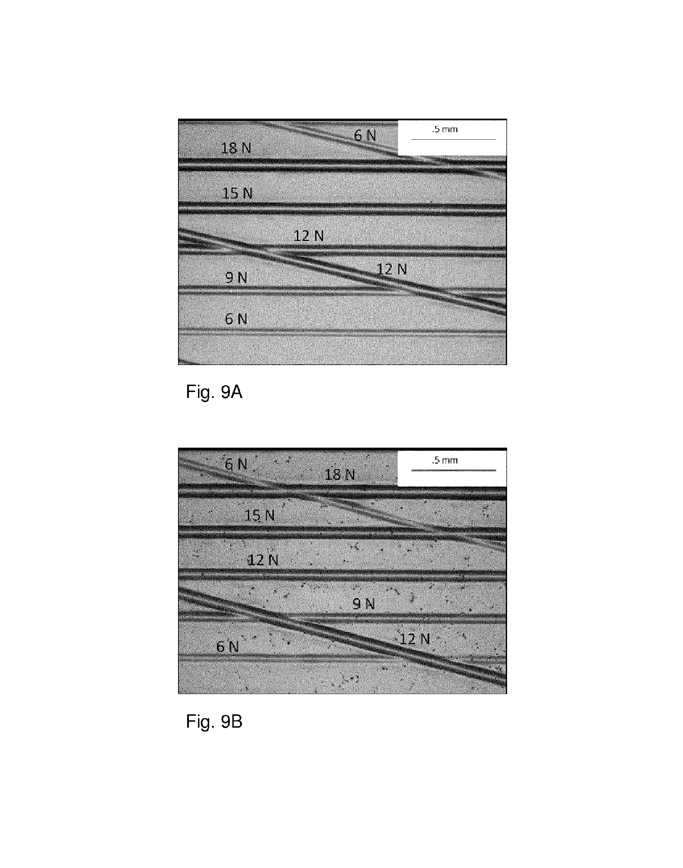

FIGS. 9A and 9B provide magnified (50×) micrograph images (in top plan view) of polished "dual-layered" ceramic coatings on (A) cast Co-28Cr-6Mo which has been pressed using a hot isostatic pressing technique homogenised, and (B) as-cast Co-28Cr-6Mo, that have been scratched with networks of five repeating groups of five parallel diamond indenter scratches were made on the corrosion test samples using a 200 µm radius diamond indenter on a scratch tester (available from CSM under the trade mark Revetest), illustrating scratches spaced 0.25 mm between centres; each group of five parallel scratches was made with scratch loads of 6, 9, 12, 15, and 18 N as shown in FIGS. 9A-9C; oblique scratches 0.75 mm apart were then made over and at a 15° angle to these parallel scratch networks at scratch loads of 6, 9, and 12 N; FIGS. 9A-9C illustrate the greater number of defects in the coating on the as-cast Co-28Cr-6Mo;
FIG. 10 depicts the results of potentiodynamic polarisation testing of scratch-damaged coating structures (scratched as described for FIGS. 9A and 9B) illustrating multi-layer CVD ceramic coatings on metal substrates which have been pressed using a hot isostatic pressing technique and homogenised, compared with as-cast metal substrates;
FIG. 11 depicts the results of potentiodynamic polarisation testing of scratch-damaged coating structures (scratched as described for FIGS. 9A and 9B) illustrating multi-layer CVD ceramic coatings compared to conventional coatings;
FIG. 12 depicts the results of potentiodynamic polarisation testing of scratch-damaged coating structures (scratched as described for FIGS. 9A and 9B) illustrating multi-layer CVD ceramic coatings on metal substrates which have been pressed using a hot isostatic pressing technique and homogenised;
FIG. 13A is an illustration of a typical Rockwell C indentation seen with multi-layer CVD ceramic coatings on metal substrates which have been pressed using a hot isostatic pressing technique and homogenised; and
FIG. 13B is an illustration of a typical Rockwell C indentation seen with a conventional CVD ceramic coatings on metal substrates which have been pressed using a hot isostatic pressing technique and homogenised.

In this document, the singular forms "a", "an", and "the" include the plural reference and reference to a particular numerical value includes at least that particular value, unless the context clearly indicates otherwise. Thus, for example, a reference to "a material" is a reference to one or more of such materials and equivalents thereof known to those skilled in the art, and so forth. When values are expressed as approximations, by use of the antecedent "about", it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive; as another example, the phrase "about 8%" preferably refers to a value of 7.2% to 8.8%, inclusive. Where present, all ranges are inclusive and combinable. For example, when a range of " 1 to 5" is recited, the recited range should be construed as including ranges "1 to 4", "1 to 3", "1 to 2", "1 to 2 and 4 to 5", "1 to 3 and 5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3 to 5, but not 2", or simply "in which 2 is not included".

In this document, chemical formulas is used as shorthand for the full chemical names. For example, "TiN" may be used to denote titanium nitride, "TiCN" to denote titanium carbonitride, "TiC" to denote titanium carbide and Al₂O₃ to denote aluminium oxide or alumina. It should be noted that the use of chemical formulas is not meant to imply that these materials are of that precise stoichiometry. In some instances, depending on deposition conditions and the like, materials may deviate from nominal stoichiometry. In addition the aluminium oxide layer can be of either kappa alumina, alpha alumina, one or more other crystalline forms of alumina, or a mixture which includes layered structures of each unless expressly limited to a particular form (although, as discussed below, alpha alumina is preferred).

The present invention relates, in part, to the discovery that the scratch, wear and corrosion resistance of a coated metal implant component can be improved or maximised by: (1) controlling the process used to prepare the metal substrate prior to coating; and/or (2) by using a coating comprising multiple "thin" layers of TiN, TiCN, or both TiN and TiCN beneath an outer layer of Al₂O₃ (preferably in the alpha form). The improvements related to the use of multiple thin layers of TiN, TiCN, or both TiN and TiCN are disclosed in US-A-2010/012926. This document provides information related to a preferred preparation of the metal substrate prior to coating. In addition, although it may be desirable to include a thicker outer layer of alumina on the coated implant in some applications, in other applications it may be desirable to use a different ceramic material for a thicker outer layer, such as a non-oxide ceramic titanium material.

As discussed above, steel alloys, cobalt, titanium and alloys containing cobalt and/or titanium are common used in the manufacture orthopaedic implants. Such materials are expected to be useful in the present invention as the metal substrate. A conventional cobalt chromium alloy useful as the metal substrate is Co-28Cr-6Mo. Co-28Cr-6Mo may be cast, wrought, forged or injection moulded, for example. For cast medical devices, Co-28Cr-6Mo may be cast according to ASTM-F75. Such a cast alloy may be used as the metal substrate for a ceramic coating. As discussed in more detail below, the as-cast Co-28Cr-6Mo may advantageously be treated by hot isostatic pressing (HIP) and homogenisation prior to coating the substrate, particularly where the outer articular surface of the ceramic coating comprises alumina. It has also been found that in some instances it may be advantageous to grind and polish the pressed (using hot isostatic pressing) and homogenised Co-28Cr-6Mo prior to coating the substrate, as discussed in more detail below.

Although the work reported below has been done with Co-28Cr-6Mo, the invention will be applicable to other cobalt chromium alloys and other metals suitable for implantation into the human body, including new materials as they are developed.

As-cast Co-28Cr-6Mo commonly has 50 to 100 µm diameter interdendritic (Co, Cr, Mo) carbides present as an inherent result of the investment casting process. When such an as-cast substrate is coated, surface carbides may increase the occurrence of defects in a ceramic coating on the substrate; for example, such surface carbides may increase the occurrence of defects in coatings including one or more layers of TiN and TiCN and an outer layer of alumina. FIG. 9B illustrates such defects, appearing as dark dots in the ceramic coating. Defects in the TiN and TiCN layers may decrease the scratch and corrosion resistance of the coating applied to the CoCrMo substrate surface, and negatively impact the cosmetic appearance of the polished coating surface.

It is also believed that the TiN and TiCN layers of an TiN/TiCN/ Al₂O₃ coating nucleate and/or grow more quickly for a given set of deposition parameters on (Co, Cr, Mo) carbides than on the solid solution matrix phase of the CoCrMo substrate. Parts of the TiN and TiCN layers above such carbides may be exposed at the surface of the coating when the Al₂O₃ layer is polished after coating. Thus, the outer surface of the polished ceramic coating may be non-homogeneous, with portions comprising alumina and adjacent portions comprising TiN, TiCN or mixtures of TiN and TiCN. These TiN and TiCN defects appear gold in colour on an otherwise brown or black polished coating surface. In such instances, the outer surface of the polished ceramic coating is defined by materials that have different properties, which may lead to sub-optimal scratch, corrosion and wear resistance of the coating.

To reduce the occurrence of TiN and TiCN defects in the outer alumina layer of the ceramic coating on the CoCrMo substrate surface, the substrate surface is preferably treated to dissolve the interdendritic (Co, Cr, Mo) carbides into the solid solution matrix phase prior to coating the substrate. This substrate surface treatment can comprise a combination of hot isostatic pressing (HIP) and homogenisation. The pressed and homogenised substrate surface is more uniform than the as-cast surface, so that the topography of the layers of ceramic coating is more even, with fewer peaks and consequently with fewer defects in the polished surface of the outer ceramic layer.

FIG. 9A illustrates a coating applied to such a surface, with fewer defects than those shown in FIG. 9B. Corrosion and scratch resistance of the coated and treated substrate is improved over the corrosion and scratch resistance of the coated as-cast substrate.

Hot isostatic pressing of the as-cast substrate may comprise, for example, placing the component in a high pressure containment vessel and pressurizing the vessel with an inert gas such as argon. The chamber is heated, resulting in pressure being applied to the component. Common pressures of the inert gas pre-heating may be, for example, between 103.4 MPa and 172.4 MPa (15,000 psi and 25,000 psi) for an as-cast Co-28Cr-6Mo substrate. Common temperatures range between 1185°C and 1204°C (2165°F and 2200°F) for an as-cast Co-28Cr-6Mo substrate. Common process times range between 4 and 4.5 hours for an as-cast Co-28Cr-6Mo substrate.

A specific example of hot isostatic pressing process parameters useful for treating an as-cast Co-28-8Mo substrate include the following: heat to 1204°C (2200°F), at a pressure of 103.4 MPa (15,000 psi) and hold at that temperature and pressure for a period of at least 4 hours.

For each of the above processes, thermocouples are used and the hold time starts when the coldest thermocouple and the minimum pressure have been obtained. In each of the above processes, the atmosphere comprises argon gas.

Homogenisation of the pressed substrate may comprise, for example, heating the pressed component to a temperature of 1204°C (2220°F) for at least four hours in an atmosphere of 500 to 700 µm partial pressure of argon, and cooling from 1204°C to 760°C (2220°F to 1400°F) in 8 minutes maximum (that is, a minimum cooling rate of from 1204°C to 760°C (2220°F to 1400°F) in 8 minutes). Appropriate homogenisation processes which might be used include processes such as surface annealing that result in the CoCr product being austenitic with a fine distribution of carbides, with no continuous blocky carbides in the grain boundaries and without widespread thermally induced porosity. Appropriate homogenisation processes which might be used include processes such as solution treatment or solutionising. Generally, the term "homogenisation" is used to refer to any process that dissolves carbide precipitates into solid solution in the metal substrate.

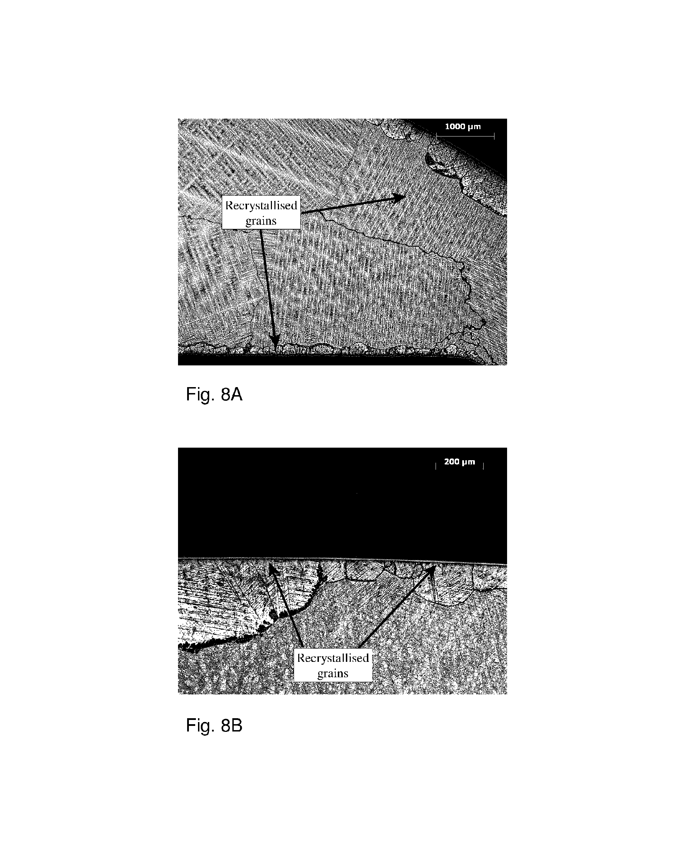

Although the pressed (using hot isostatic pressing) and homogenised metal substrate may then be coated with ceramic material (including multiple layers of ceramic material), if the metal substrate has been mechanically worked prior to pressing and homogenizing, the inventors of the present invention have discovered that when a mechanically-worked pressed and homogenised Co-28Cr-6Mo metal substrate is subsequently coated with layers of TiN, TiCN, combinations of TiN and TiCN and Al₂O₃, adhesion of some of the layers of the coating to the metal substrate may be less than optimal. The inventors discovered that the pressed and homogenised Co-28Cr-6Mo that had been rough ground and CNC (computer numerical control) ground prior to the pressing and homogenisation treatments had recrystallised grains present on the surface of the metal substrate (such grains are illustrated in FIGS. 8A and 8B). Such recrystallised grains may also result from other processes that involve mechanical working of the metal substrate, such as shot peening to clean the as-cast component. If the metal substrate is mechanically worked prior to pressing and homogenizing, the metal substrate is preferably machined or ground after pressing and homogenisation steps to remove recrystallised grains. The inventors discovered that the bond between the metal substrate and the surface coating can be improved by performing a rough and CNC grinding step after the metal substrate has been pressed and homogenised.

In general, if about 0.5 to 1 mm of the outer surface of the pressed (using hot isostatic pressing) and homogenised substrate is removed through a grinding, machining, polishing or other mechanical process, recrystallised grains should be removed, leaving the parent phase of the metal on the substrate surface, providing a better surface to receive and bond with the ceramic coating. Any available machining, grinding or polishing technique and equipment that removes this amount of material from the outer surface of the substrate should suffice for the purposes of this process. The ground/machined surface is preferably polished to a mirror smooth finish (for example, having a surface roughness Ra of 0.03 or 0.04 µm prior to coating; see ISO 4287 (1997)).

The presence of recrystallised grains in the metal substrate appears to decrease the growth rate of the ceramic coating on the metal substrate. For a fixed process time for forming the coating, the thickness of at least the initial layers of the ceramic coating may thus be reduced, seeming to lead to a weaker bond with the outer alumina layer. Accordingly, instead of removing a portion of the outer surface of the pressed and homogenised metal substrate, it is expected that the adverse effect of recrystallised grains could be reduced by adjusting the process parameters for forming the initial layers of the ceramic coating, such as by increasing the process time for forming the initial layer or layers.

The pressing processes discussed above which use hot isostatic pressing may also advantageously close internal porosity of the as-cast metal substrate.

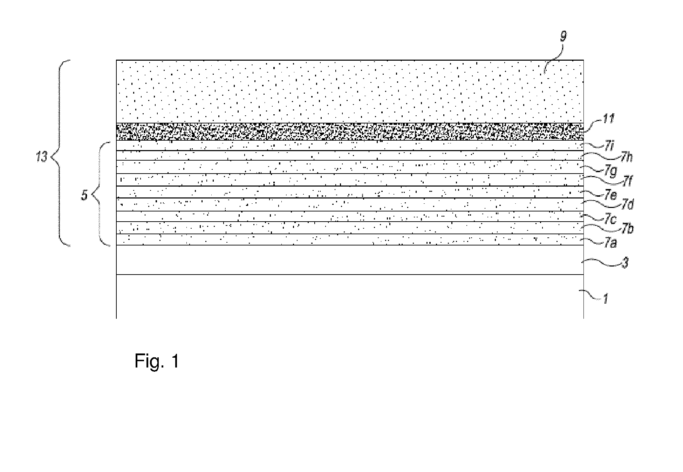

The pressed, homogenised and ground/machined/polished substrate may then be coated with ceramic material. The ceramic coating and technique may produce a dual layer coating, as described in US-A-2007/0078521. Alternatively, the pressed, homogenised and ground/machined/polished substrate may then be coated with multiple thin layers of ceramic as described in US-A-2010/0129626. The ceramic coating may comprise three stacked bands (shown diagrammatically in cross-section in FIG. 1) overlying the metal substrate 1: a first band or region 3 comprising a first ceramic layer formed upon the metal substrate 1; a second band or region 5 comprising multiple thin ceramic layers 7 formed upon the first band 3; and a top or outer band or region 9 comprising a thicker layer of ceramic. A fourth band or region 11, comprising a bonding band, may be provided between the second band 5 and the top or outer band 9; the bonding band may comprise a single layer of ceramic material to improve the bonding between the outermost layer 7 of the middle band 5 and the top or outer band 9 of the ceramic coating. The outer surface of the outermost band may be polished to define the articular surface of the finished orthopaedic implant component.

Preferably, the first band 3 or first layer comprises TiN, TiCN, or both TiN and TiCN is deposited upon the pressed (by hot isostatic pressing), homogenised and ground/machined/polished metal substrate 1, followed by the middle band 5, comprising multiple thin layers (e.g. 7a-7i) of TiN, TiCN or both TiN and TiCN deposited on the first band or layer 3 and followed by the thicker outer band 9 comprising a single layer of ceramic material deposited on the outermost layer of the middle band 5.

The layers defining the first 3 and middle 5 ceramic bands may comprise TiN, TiCN, or both TiN and TiCN. Where the first band/layer 3 comprises one of TiN, TiCN or both TiN and TiCN, the initial layer 7a of the middle band 5 preferably comprises a different one of TiN, TiCN or both TiN and TiCN. The subsequent layers 7b et seq. of the middle band 5 may comprise one or more repetitions of the first band/layer 3 and layers 7a et seq. of the middle band 5. As used herein, a layer that is a "repetition" of a different layer is generally of the same chemical composition as the different layer, of the same thickness as the different layer, or both. For example, if the first band/layer 3 includes only TiN and the adjacent layer 7a includes only TiCN, two subsequent layers 7b, 7c that are repetitions of these layers 3, 7a will include only TiN and TiCN, respectively. The entirety of the complement of subsequent layers 7b et seq. may comprise one or more repetitions of the first and second layers 3, 7a, or only some of the subsequent layers 7b et seq. may comprise one or more repetitions of the first and second layers 3, 7a. In one embodiment, the second layer 7a is different than the first layer 3, and all of the subsequent layers 7b et seq. comprise repetitions of the first and second layers 3, 7a; the resulting structure will therefore comprise layers that alternate between the material of the first layer 3 and the material of the second layer 7a. In a preferred version of this embodiment, the first layer 3 is TiN, the second layer 7a is TiCN, and the subsequent layers 7b et seq. comprise alternating layers of TiN and TiCN. Among the first layer 3, the second layer 7a, and the at least one subsequent layer 7b et seq., it is preferred that at least one of the layers comprises TiN and at least one adjacent layer comprises TiCN. The top or final layer of the middle band 5, i.e., the last of the at least one subsequent layers, may comprise TiCN, TiN or a mixture TiN and TiCN.

The material used for the top or outer ceramic band or layer 9 may vary depending on the anticipated bearing environment. For example, if the implant component is expected to bear against a polymer such as ultrahigh molecular weight polyethylene, then the top or outer ceramic band or layer 9 may preferably comprise alumina. If the component is expected to bear against a different material, such as another ceramic-coated metal substrate or a harder material like metal or another ceramic, and to thus be placed in high contact stress applications, the top or outer ceramic band or layer 9 may comprise TiN, TiCN or a mixture of TiN and TiCN instead of alumina.

It should be understood that it is anticipated that other ceramic materials may be useful in the present invention. For example, it is anticipated that titanium carbide TiC could be used as part of the ceramic coating.

The thickness of the first band or layer 3 may be less than about 10 µm, less than about 8 µm, less than about 6 µm, less than about 5 µm, 2 to 3 µm or about 2.5 µm. Preferably, the thickness of the first band or layer 3 is about 2to 3 µm, and most preferably, about 2.5 µm. Generally, the first ceramic band 3 comprises a ceramic layer that is thicker than the individual ceramic layers 7 of the second ceramic band 5.

The middle ceramic band 5 may comprise multiple thin ceramic layers 7 deposited upon the first ceramic band or layer 3. The initial layer 7a of the middle ceramic band 5 may have a thickness that is less than about 1 micron, less than about 0.75 µm, less than about 0.5 µm, less than about 0.3 µm, less than about 0.2 µm, or less than about 0.1 micron. In some embodiments, the initial layer 7a of the second band 5 may have a thickness that is about 0.1 µm, about 0.2 µm, about 0.3 µm, about 0.5 µm, about 0.7 µm, about 0.8 µm, about 0.9 µm, about1 micron, about 2 µm, about 3 µm, about 4 µm and about 5 µm. The initial layer 7a of the middle band 5 preferably has a thickness that is less than that of the first band/layer 3. The middle ceramic band preferably includes multiple thin layers of ceramic, and may include, for example, 2 to 100 thin layers of ceramic, 2 to 50 thin layers of ceramic, 5 to 50 layers of ceramic, 10 to 50 layers of ceramic, 20 to 50 layers of ceramic, or 30 to 50 layers of ceramic. As illustrated below, improved scratch resistance can be achieved with about 30 layers of ceramic as well as with about 50 layers of ceramic in the middle band 5.

Alternatively, the second band 5 may comprise a single ceramic layer deposited upon the first ceramic band or layer 3. For example, the second band 5 may comprise a single layer of TiN, TiCN or a mixture of TiN and TiCN having a thickness of about 2.5 µm, although it is believed that optimum results are achieved when the second band comprises multiple thinner layers of ceramic.

The top or outer ceramic band 9 preferably comprises a single thicker layer of ceramic material. The top or outer ceramic band may, for example, be alumina having a thickness of about 2 µm to 15 µm, for example, about 3 µm to 15 µm, about 4 µm to about 15 µm, about 5 µm to 15 µm. In a particular embodiment, the top or outer ceramic band 9 is about 4 to 7 µm thick. The top or outer ceramic band may comprise, for example, Al₂O₃, TiN, TiCN or both TiN and TiCN and have a thickness of about 1 µm, about 2 µm, about 3 µm, about 4 µm, about 5 µm, about 6 µm, about 7 µm, about 8 µm, about 9 µm, about 10 µm, about 12 µm, about 15 µm, about 17 µm or about 20 µm. In some embodiments, the top or outer ceramic band is the thickest of the all the layers defining the ceramic coating. The thickness of the outermost layer may be dictated by any of a number of considerations readily understood among those skilled in the art, such as production cost, implant type, environment of use, layer adhesion, inherent layer durability, the roughness of the as-deposited outermost layer and the need to polish the coating, and the like.

Preferably, the entire ceramic coating 13 (including the first, second and third bands 3, 5, 9 and any bonding band 11) has a thickness of between about 8.5 µm and 20 µm, and more particularly, between about 8.5 µm and about 15 to 16 µm. It has been found that if the ceramic coating is too thick, then the coating may fail mechanically.

Examples of thicknesses for the ceramic coating are set forth in the table below:

| Band | Layer | Preferred thickness |
|---|---|---|
| First band | 1 (against metal substrate) | From >1 µm to <3 µm, preferably 2.5 µm |
| Middle band | 2-100 | From about 0.1 µm to <1 µm per layer, with an overall thickness of the middle band being about 3 to 5 µm |
| Top band | Top/outermost layer | From 2 to 15 µm, preferably 5.5 µm |

The term "thickness" when used in relation to a given layer or the entire ceramic coating refers to the average thickness of that layer or coating over its entire area; accordingly, if the "thickness" of a layer is about 1 µm, there may be portions of that layer that are less than 1 µm thick, and/or portions of that layer that are thicker than 1 µm, but the average thickness over the entire area of the layer may be calculated as about 1 µm.

The metal substrate preparation processes of the present invention are expected to be most advantageous when used in conjunction with a chemical vapour deposition (CVD) process for forming the bands and layers of ceramic on the metal substrate. It is expected that once provided with the desired number, constituency and thickness of the layers defining the coating, those in the coating art (such as Ionbond AG Olten, of Olten, Switzerland, Seco Tools AB, of Fagerstra, Sweden, and Sandvik AB of Sandvik, Sweden) will readily set CVD process parameters (such as temperature, pressure, reactive concentrations and heating and cooling rates) to deposit the layers as desired.

It should be understood, however, that processes other than CVD processes can be used to deposit the bands or layers. Various techniques (such as physical vapour deposition, chemical vapor deposition, and thermal spraying deposition, for example, plasma spraying) are available for forming bands and layers of ceramic coatings, although it may be difficult to form some of the thinner layers using plasma spraying. According to the invention, the deposition of any of the layers may be performed in accordance with any acceptable technique that provides layers having desired characteristics (for example thickness profile). Although the respective bands and layers may all be deposited using a single technique, it is anticipated that different bands and layers may be deposited using different techniques; for example, thicker layers may be deposited by a technique that is suitable for "thick" layer deposition, whereas thinner layers may be deposited by a technique that may achieve deposition of thinner layers. The advantages of the metal substrate preparation processes of the invention may be expected to vary somewhat with the technique used to deposit the ceramic coating, particularly with the technique used to deposit the first band adjacent to the substrate.

The process or method of the present invention may also include depositing a bonding band or layer (band 11 in FIG. 1) upon the at least one subsequent layer prior to depositing the at least one layer that comprises aluminium oxide (e.g. layer 9 in FIG. 1). In other words, a bonding layer 11 may be deposited upon the last/outermost layer (e.g. layer 7i in FIG. 1) of the middle band 5. Such bonding layers are also known as alumina bonding layers, oxide bonding layers, or kappa or alpha nucleation layers and have been previously described for use in increasing the bonding strength between an aluminium oxide layer and an adjacent material and/or to promote the formation of the desired aluminium oxide crystalline phase. Bonding layers between aluminium oxide and an adjacent material that may be used pursuant to the present invention are described, for example, in US-4463062, US-6156383, US-7094447, and US-A-2005/0191408, and in the paper by Zhi-Jie Liu et al entitled "Investigations of the bonding layer in commercial CVD coated cemented carbide inserts", Surface & Coatings Technology 198 (2005) 161- 164. The bonding layer may comprise one or more of an oxide, an oxycarbide, an oxynitride, and an oxycarbonitride of a metal from Group IVa, Va, or VIa of the periodic table of the elements. For example, the bonding layer may comprise one or more of a titanium oxide, a titanium oxycarbide, a titanium oxynitride, and a titanium oxycarbonitride. It is anticipated that Cr₂O₃ may be used as a bonding layer or template for PVD deposition of α-alumina. In some embodiments the bonding layer may be a mixture of materials, such as a mixture of oxides, for example, a mixture of titanium oxides.

Available techniques for depositing a bonding layer will be appreciated by those skilled in the art, such as any of the techniques describe above with respect to the deposition of the first, second, and at least one subsequent layers. For example, chemical vapor deposition may be used to deposit a bonding layer in accordance with the present invention. Bonding layers may have a thickness that is less than 2 µm, and may have a thickness less than 1 µm, less than 500 nm, less than 250 nm, less than 100 nm, less than 50 nm, less than 30 nm, less than 20 nm, or less than 10 nm. Various companies (for example, Ionbond AG Olten, of Olten, Switzerland, Seco Tools AB, of Fagerstra, Sweden, and Sandvik AB of Sandvik, Sweden) provide the service of applying bonding layers and can be contacted for this purpose.

It is expected that the constituency and thicknesses of the layers defining the coating of a finished component may be analysed using known techniques, such as TEM (transmission electron microscopy, no less than 10,000 magnification), EDX Energy-dispersive X-ray spectroscopy or EELS (Electron energy loss spectroscopy).

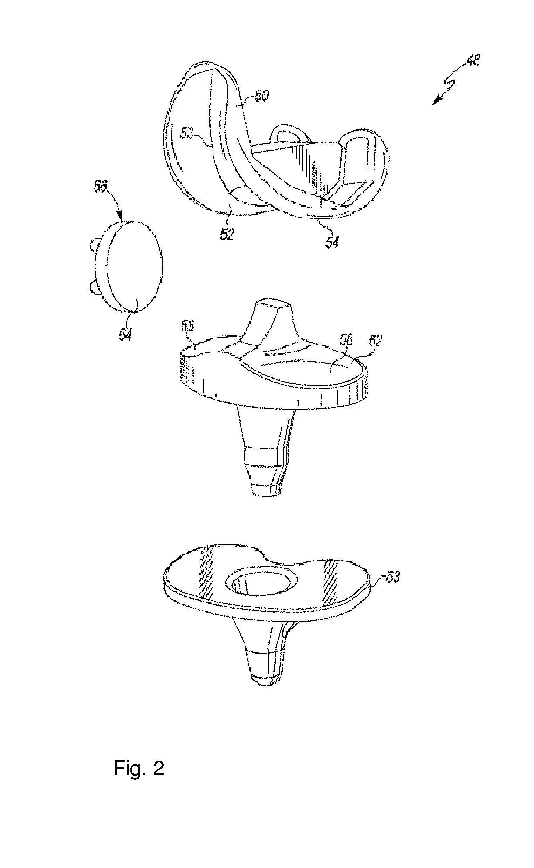

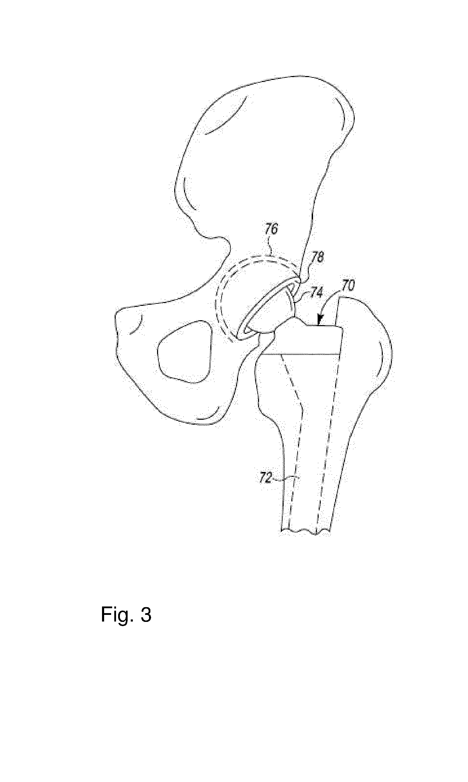

FIGS. 2 and 3 illustrate examples of orthopaedic implant components that may be produced using the principles of the present invention. For example, FIG. 2 illustrates a knee implant system 48 in which the distal femoral component 50 has articulation surfaces 52, 53, 54 that are designed to bear against the articulation surfaces 56, 58, of a tibial bearing 62 (that is received on a tibial base 63) and an articulation surface 64 a patellar implant component 66. In such an environment, one might expect the tibial bearing 62 and bearing surface 64 of the patellar implant component 66 to comprise ultra high molecular weight polyethylene stabilised against oxidation. The articulating surfaces 52, 53, 54 of the femoral component 50 (including both condylar articulating surfaces 52, 53 and the intercondylar groove 54) may be pressed (using hot isostatic pressing), homogenised, ground/machined/polished and ceramic coated as described above. For an such implant system using a polyethylene bearing component, the top or outer layer of the ceramic coating may advantageously comprise alumina.

FIG. 3 illustrates a hip implant system 70 including a proximal femoral stem 72 with an articulating ball 74 at the proximal end of the stem 72, an acetabular cup 76 and an acetabular bearing insert 78. Typical materials for the bearing inserts 78 include ultra high molecular weight polyethylene, metal (cobalt chromium alloy, for example) or ceramic. The entire outer surface of the articulating ball 74 at the proximal end of the stem 72 may advantageously be pressed (using hot isostatic pressing), homogenised, ground/machined/ polished and ceramic coated as described above. If the ball 74 is designed to articulate against a polymer bearing (such as UHMWPE), the top or outer band or layer may comprise alumina. If the ball 74 is designed to operate in a high contact stress environment (that is, to articulate against a hard bearing component such as metal or ceramic rather than a polymeric bearing component), then it may be desirable to use a different material for the top or outer layer, such as TiN, TiCN or both TiN and TiCN, that provides optimum performance against another hard surface.

Orthopaedic implants such as those shown in FIGS. 2 and 3, as well as other articulating orthopaedic implant systems (such as shoulder implant systems and ankle implant systems) treated and coated in accordance with the present invention are expected to have advantageous properties: improved adhesion of the coating to the substrate, and improved resistance to corrosion, scratching and wear. It should be understood that the coating may be applied to select portions of the outer surface of the implant component or to the entire outer surface of the implant component; for example, the portion of the outer surface of the implant component that bears or articulates against a portion of another component may be selectively coated.

It should be understood that knee, hip, shoulder and ankle orthopaedic implant components may be provided in the form of kits. For example, a knee implant kit may include all of the elements illustrated in FIG. 2 in varying sizes to suit the particular patient and a hip implant kit may include all of the elements shown in FIG. 3 in varying sizes to suit the needs of a particular patient.

Particular processes used in preparing samples and particular tests run on at least some of these samples are described below. Unless otherwise indicated, the samples were prepared from flat discs, rather than from implant components.

### Metal Substrate Preparation

Samples of as-cast Co-28Cr-6Mo cobalt chromium alloy were obtained as well as a sample of a Zr-Nb alloy and a sample of a titanium alloy. The following table summarises the material and initial preparation parameters for the metal substrates.

| Metal substrate preparation | |
|---|---|
| Sample | Material and actions performed |
| 1 - 8, 16-17, 20-24 | Cast Co-28Cr-6Mo, shot peen/grit blast to remove scale, grind, polish metal substrate |
| 7 | Mill-annealed Ti-6A1-4V bar stock, grind, polish metal substrate |
| 8 | Zircadyne 705 (Zr-2.5Nb) bar stock, grind, polish metal substrate, thermal oxidise in air at 500°C for 4 hours, polish ZrO₂ surface |
| 6, 9-15, 18-19 | Cast Co-28Cr-6Mo, shot peen/grit blast to remove scale |

The following tables summarise the further processes used in preparing the metal substrates prior to coating.

| Metal substrate preparation - heat treatments | | | | | | |
|---|---|---|---|---|---|---|
| Sample | Hot isostatic pressing | | | Homogenisation | | |
| | Temp | Time | Pressure | Temp | Time | Pressure |
| 1-8, 14-15, 18-19 | None | | | | | |
| 9-13, 16-17, 20-24 | 1185°C (2165°F) | 4.0-4.2 h | 172 MPa (25 ksi) Ar | 1216°C (2220°F) | 4.0-4.2 h | Vacuum^{a} |

| | | | | | | |
|---|---|---|---|---|---|---|
| ^{a} The chamber was evacuated to a partial pressure of 500 to 700 µm argon | | | | | | |

| Metal substrate preparation - post-heat treatment and pre-coating steps | |
|---|---|
| Sample | Action performed, stage action performed |
| 1-8, 14-15, 18-19 | None |
| 8 | Thermal oxidise in air at 500°C for 4 hours |
| 9-13, 16-17, 20-24 | Grind, polish metal substrate after pressing and homogenizing |

### Coating

Coatings were applied by lonbond AG Olten of Olten, Switzerland.

The table below summarises the characteristics of the coatings applied to the samples.

| Sample | Coating | | |
|---|---|---|---|
| | Inner band | Second band | Outer band |
| 1-5, 18-19 | TiN (single layer, 2.3 µm; CVD) | 50 alternating layers of TiCN and TiN (each layer about 0.1 µm to a total thickness of 5.5 µm; CVD) | Al₂O₃ (5 µm) |
| 6, 14-17 | TiN (single layer, 2.5 µm; CVD) | TiCN (single layer, 2.5 µm) | Al₂O₃ (5 µm) |
| 7 | TiN only (single layer, 10 µm; PVD) | None | None |
| 8 | Oxidised Zr-Nb | None | None |
| 9-13,20-21 | TiN (single layer, 1.5 µm; CVD) | 50 alternating layers of TiCN and TiN (each layer about 0.1 µm to a total thickness of 5.5 µm; CVD) | Al₂O₃ (5 µm; CVD) |
| 22 | TiN (single layer, 2.5 µm; CVD) | 50 alternating layers of TiCN and TiN (each layer 0.1 µm to a total thickness of 5 µm; CVD) | Al₂O₃ (5 µm; CVD) |
| 23-24 | TiN (single layer, 1.5 µm; CVD) | 36 alternating layers of TiCN and TiN (each layer about 0.1 µm to a total thickness of about 3.5 µm; CVD) | Al₂O₃ (5 µm; CVD) |

Most of the ceramic-coated samples were polished prior to testing; samples 14, 16, 18 and 20 were not polished before testing.

### Scratch Testing

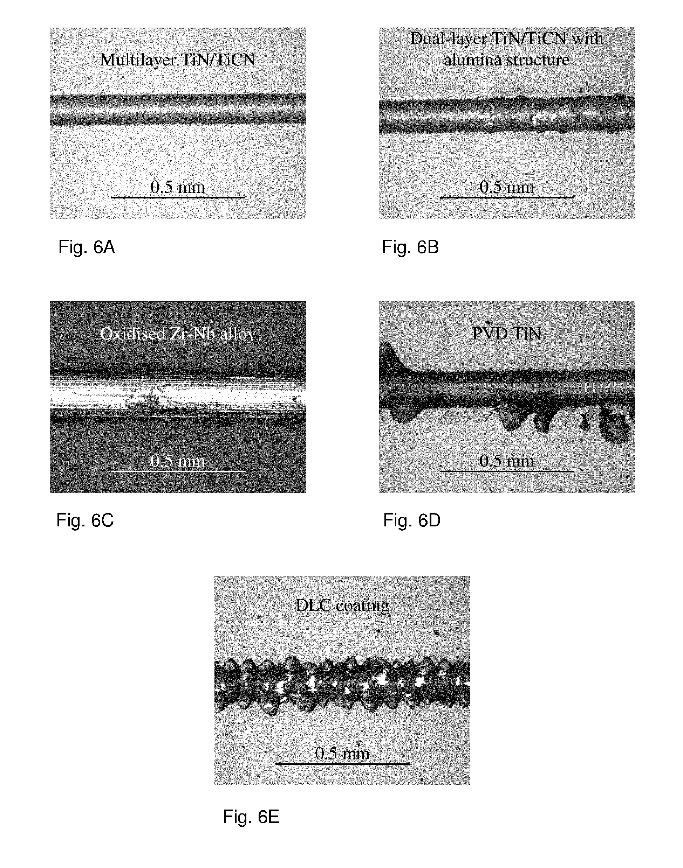

To compare the resistance of conventional coatings and the present coatings to surface damage by scratching, 10 mm long scratches were formed along the surface of coated samples 6, 7, 8 and 9 using a 200 µm radius diamond indenter tip on a CSM Revetest scratch tester under a constant load of 40 N (a relatively high load compared to scratching loads that would be expected to act on an implant that has been implanted in a patient), using the techniques discussed in the paper by Smith, B et al "Pin on Disc Wear Testing of a Scratched Engineered Surface", Transactions 55th ORS, No. 2292 (2009). FIGS. 6A to 6E show the results of this scratch test. The sample in FIG. 6A corresponds to Sample 9 in the above tables; the sample in FIG. 6B corresponds to Sample 6 in the above tables; the sample in FIG. 6C corresponds to Sample 8 in the above tables; the sample in FIG. 6D corresponds to Sample 7 in the above tables; the sample in FIG. 6E is not shown in the above tables.

As depicted in FIGS. 6A to 6E, the results of the test revealed superior mechanical performance of the sample (Sample 9) that was pressed, homogenised, ground, polished and then coated with three bands of ceramic, including a middle band having 50 thin layers of ceramic.

With respect to the structure of Sample 6 (a single, 2.5 µm thick inner band of TiN, a second band comprising a single, 2.5 µm thick layer of TiCN, and an outer band comprising 5 µm thick alumina overlayer; such structures are generally referred to as "dual layered" herein in reference to the total number of layers of TiN and TiCN), it was observed that cracks and alumina spalls (Lc2-type cracking according to ASTM C1624-05 specification) occurred at regular intervals along the scratch length (FIG. 6B), whereas there were no Lc2-type cracks observed in the structure of Sample 9 (FIG. 6A).

Scratching of the oxidised Zr-Nb alloy of Sample 8 (5 µm oxide layer) at such relatively high loads (Zr is relatively soft) resulted in exposure of the base substrate material within the scratch trough along the entire length of the test damage (FIG. 6C; substrate material visible as a white line at the centre of the scratch).

The images of the monolayer TiN coating of Sample 7 (thickness 10 µm, deposited by arc evaporation PVD) on a Ti-6A1-4V substrate show that large chips of the coating material were removed along the scratch line, exposing the substrate material (FIG. 6D).

The diamond-like carbon (DLC) coating (available from Richter Precision Inc under the trade mark Medikote C11), thickness 6 µm, deposited by PVD on pressed/ homogenised F75 CoCrMo substrate; not shown in the above tables) underwent considerable chipping under 40 N applied loads (FIG. 6E).

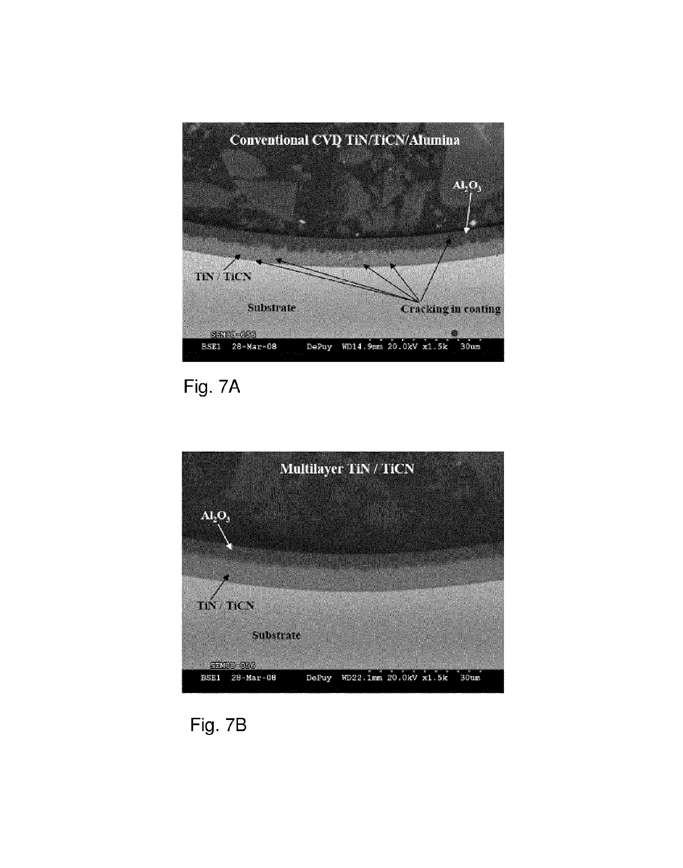

FIG. 7 provides magnified images from a scanning electron microscope (SEM) analysis of polished cross sections of conventional coatings and coatings as provided by the invention through 40 N constant load scratches. It is apparent that the coating of Sample 9 (FIG. 7B) is far less susceptible to microcracking within the TiN/TiCN layers under the alumina overlayer than the "conventional CVD" structure of Sample 6, in which cracks and fissures are observed within the TiN and TiCN monolayers (FIG. 7A).

In addition to the optical analysis of the scratches, scratches were also analysed acoustically to determine the number of acoustic emission peaks characteristic of Lc2 chipping or buckling spallation type cracking events (according to ASTM C 1624-05) that occur under various load conditions. The polished samples where polished using Buehler Metadi diamond suspensions together with Texmet papers; polishing was undertaken starting with a 9 µm diamond suspension, through a 6 micron diamond suspension and ending with a 1 µm diamond suspension. Polished samples were polished to an optically flat finish. Five scratches were created under constant loads of 20 N, 25 N, 28 N, 30 N and 40 N, positioned 0.25 mm apart, each scratch 10 µm in length, and performed at a speed of 1 mm per second, using a 200 µm radius diamond indenter tip on a scratch tester (available from CSM under the trade mark Revetest). Results are as follows:

| Sample | Number of acoustic emission peaks characteristic of Lc2 chipping or bucking spallation per mm of scratch length - measured by acoustic emission and supported by optical inspection | | | | |
|---|---|---|---|---|---|
| | 20 N | 25 N | 28 N | 30 N | 40 N |
| 14 - as coated | 0.3 | 0.8 | 1.1 | 1.6 | 6.8 |
| 15 - polished | 0.1 | 0.8 | 1.0 | 1.4 | 6.5 |
| 16 - as coated | 0.3 | 0.6 | 1.6 | 1.8 | 6.0 |
| 17 - polished | 0.2 | 0.7 | 1.4 | 1.9 | 5.8 |
| 18 - as coated | - | - | - | - | - |
| 19 - polished | 0.1 | 0.3 | 0.8 | 1.5 | 5.8 |
| 20 - as coated | 0 | 0 | 0 | 0 | 0.8 |
| 21 - polished | 0 | 0 | 0 | 0 | 1.0 |
| 22 - polished | - | 0 | - | 0 | 0.02^{a} |
| 23 - polished | - | - | - | 0 | 0.5 |
| 24 - polished | - | - | - | 0 | 0.8 |

| | | | | | |
|---|---|---|---|---|---|
| ^{a} The value was 1.0 when the scratch length was 50 mm | | | | | |

Progressive load scratch testing of various samples was also performed according to ASTM C1624-05 using a 200 µm radius diamond indenter tip using the CSM Revetest scratch tester. The dual layer samples comprised an inner band and a middle band; these two bands comprised a layer of TiN and a layer of TiCN; these two layers were covered with an outer band comprising an Al₂O₃ overcoat. The multilayer coating samples all had a middle band comprising 50 layers of TiN and TiCN coated onto a single layer inner band TiN and an Al₂O₃ overcoat as the outer band. The results are presented in the table below:

| Sample | Average load (N) | | |
|---|---|---|---|
| | Lc1 | Lc2 | Lc3 |
| Conventional dual layer TiN/TiCN/Al₂O₃ on as cast Co-28Cr-Mo | 10.1 | 37.2 | 80 |
| Conventional dual layer TiN/TiCN/Al₂O₃ on pressed and homogenised Co-28Cr-Mo | 10.8 | 37.8 | 82 |
| Multilayer (50) TiN/TiCN/Al₂O₃ on as cast Co-28Cr-Mo | 9.2 | 39.7 | - |
| Multilayer (50) TiN/TiCN/Al₂O₃ on pressed and homogenised Co-28Cr-Mo | 12.5 | 45 | 95 |

These results demonstrate that multilayer coatings which have been pressed, homogenised, ground and polished according to the present invention minimise scratch-induced damage and are more effective in preventing the generation of microcracks as compared with conventional coatings.

Samples 1 to 13 were aggressively scratched in preparation for corrosion testing. For these samples, networks of five repeating groups of five parallel diamond indenter scratches were made on the corrosion test samples using a 200 µm radius diamond indenter on a CSM Revetest scratch tester. The scratches were spaced 0.25 mm between centres. Each group of five parallel scratches was made with scratch loads of 6, 9, 12, 15, and 18 N as shown in FIGS. 9A and 9B. Oblique scratches 0.75 mm apart were then made over and at a 15° angle to these parallel scratch networks at scratch loads of 6, 9, and 12 N. Representative micrographs (50× magnification) of these scratch networks are shown in FIGS. 9A and 9B. These samples were then corrosion tested as described below.

### Corrosion Testing

All samples subjected to corrosion testing (Samples 1 to 13) first had scratch networks put onto the outer surface of the ceramic coating as described above.

Cyclic potentiodynamic polarisation testing was performed on some samples. The testing method was similar that described in ASTM F2129. A BioLogic VMP3 potentiostat/galvanostat with a flat cell and a saturated Ag/AgCl/KCl reference electrode was used. Some of the samples were scratched and cyclic scanned in Hanks solution with 25 vol.% bovine calf serum at 37°C to simulate the presence of biological macromolecules and increased viscosity conditions *in vivo*. Cyclic polarisation testing was performed on polished and scratched samples was to measure the corrosion resistance of the coating/ substrate system in the presence of simulated excessive *in vivo* abrasive scratch damage.

The test area of each sample was immersed in electrolyte for 1 hour prior to each scan to allow open circuit potential stabilisation. Cyclic polarisation scans were performed at a scan rate of 0.166 mV.s⁻¹ (10 mV.min⁻¹).

The solution used was HyClone HyQ Hanks solution (Part no. SH30030) (see below), mixed with 25 vol.% HyClone bovine calf serum (Part no. SH30073.03). The solution was not deaired. It had a pH of 7.4 prior to or during any of the scans. In the cyclic potentiodynamic scans, the test area of each sample was immersed for 1 hour prior to each scan to allow open circuit potential stabilisation.

The chemical composition of HyClone HyQ Hanks balanced salt solution SH30030 liquid was as follows:

| Component | mg.l⁻¹ |
|---|---|
| KCl | 400 |
| KH₂PO₄ | 60 |
| MgSO₄ | 97.67 |
| NaCl | 8000 |
| Na₂HPO₄ | 47.68 |
| CaCl₂ | 140 |
| D-glucose | 1000 |
| Phenol red | 11 |
| NaHCO₃ | 350 |
| Water | Balance |

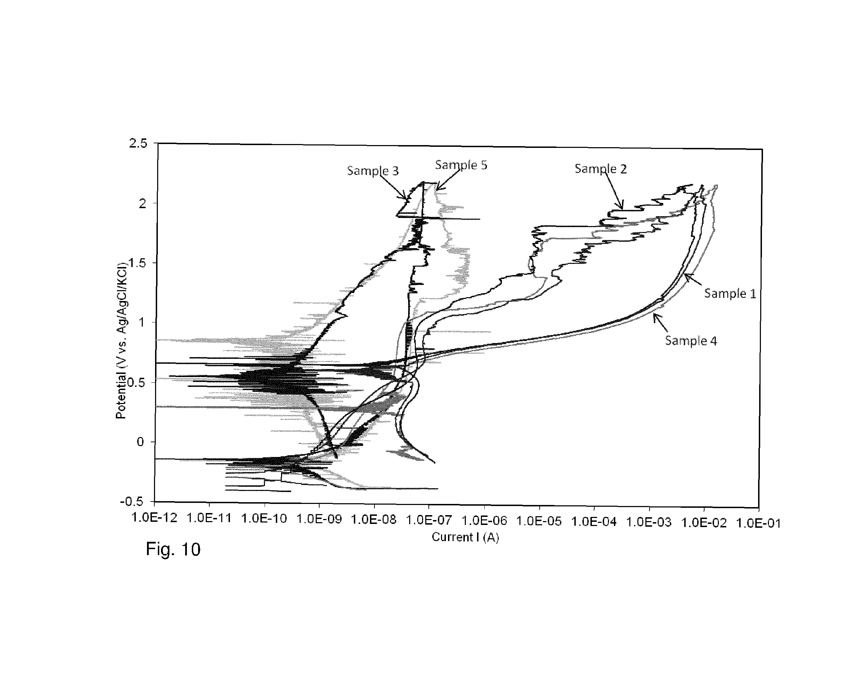

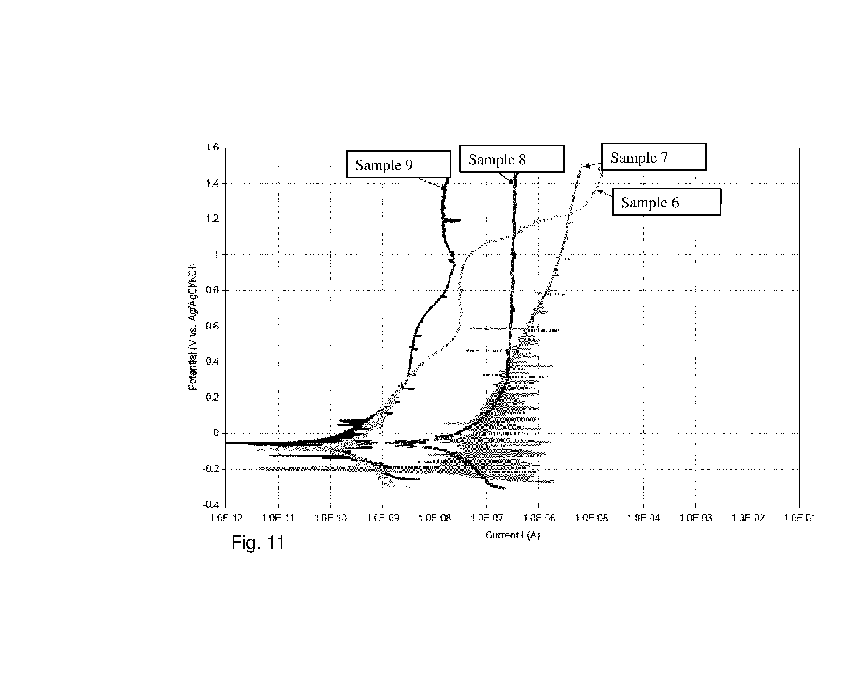

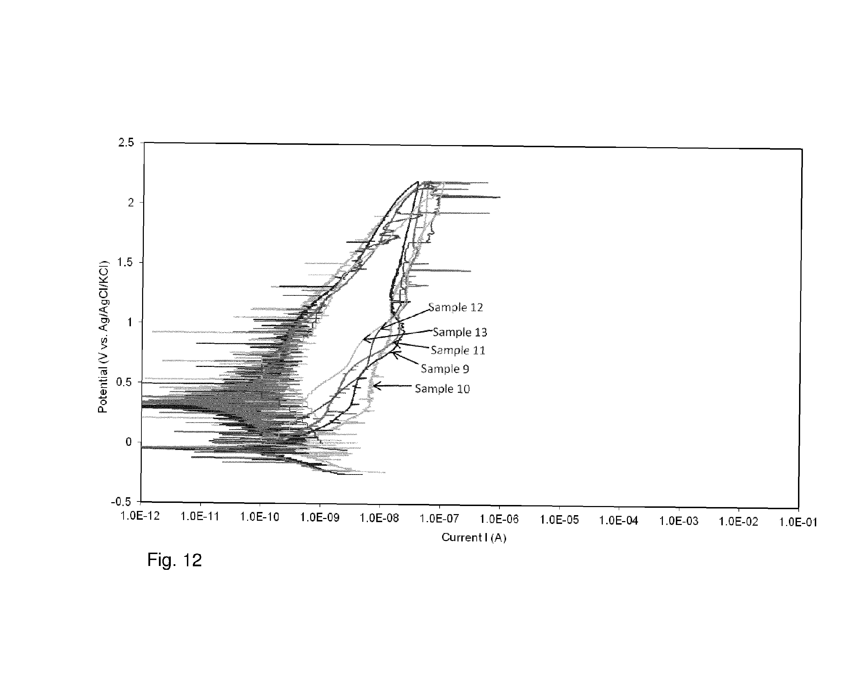

The results of the cyclic potentiodynamic polarisation testing are shown in FIGS. 10 to 12, with samples 1 to 5 shown in FIG. 10, samples 6 to 9 shown in FIG. 11, and samples 9 to 13 shown in FIG. 12.

It can be seen from FIG. 11 that sample 9 had lower current levels compared to zirconia (Sample 8), 2 layer TiN/TiCN (Sample 6) and PVD TiN (Sample 7). However, as can be seen from FIG. 10, results varied substantially with TiN/TiCN samples coated onto an as-cast metal substrate. In comparison, FIG. 12 shows that the breakdown current became much more consistent for multi-layer samples that had been pressed using hot isostatic pressing and homogenised. Taken together, the cyclic potentiodynamic polarisation testing shows that the multi-layer samples are consistently more corrosion resistant when the substrates are pressed, homogenised and then ground before coating and that the pressed/homogenised/ground/multilayer samples were more corrosion resistant than the conventional dual layer sample.

### Rockwell C Indentation

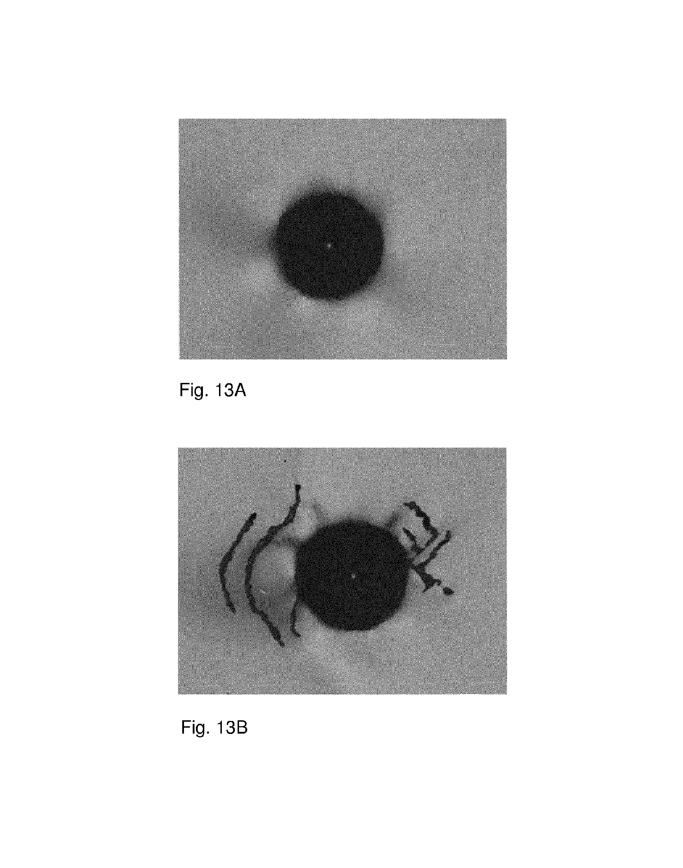

Rockwell C indentation was performed on polished areas of coated samples consistent with the VDI 3198 standard. Hardness was measured and deformation patterns in the samples were optically analysed to detect any spalling/cracking around the indentation marks. Hardness values were consistently between about 36 and 40 RHC, the measured value for the substrate material. A comparison of FIGS. 13A and 13B shows that much less cracking and chipping of the coating occurs at the periphery of the indentations with the multilayer coating as compared to the conventional dual layer coating. See Vidakis, N et al, "The VDI 3198 Indentation Test Evaluation of a Reliable Qualitative Control for Layered Compounds", Journal of Materials Processing Technology 143-144 (2003), pp 481-485. Both samples comprised Co-28Cr-Mo that had been pressed using hot isostatic pressing and homogenised and then coated with ceramic. The sample shown in FIG. 13A comprises a ceramic coating on this substrate, the coating comprising an inner band of TiN, a middle band comprising multiple thin alternating layers of TiN and TiCN, and an Al₂O₃ overcoat. The sample shown in FIG. 13B comprises a ceramic coating on this substrate, the coating comprising two layers of TiN and TiCN with an Al₂O₃ overcoat.

### TEM Imaging

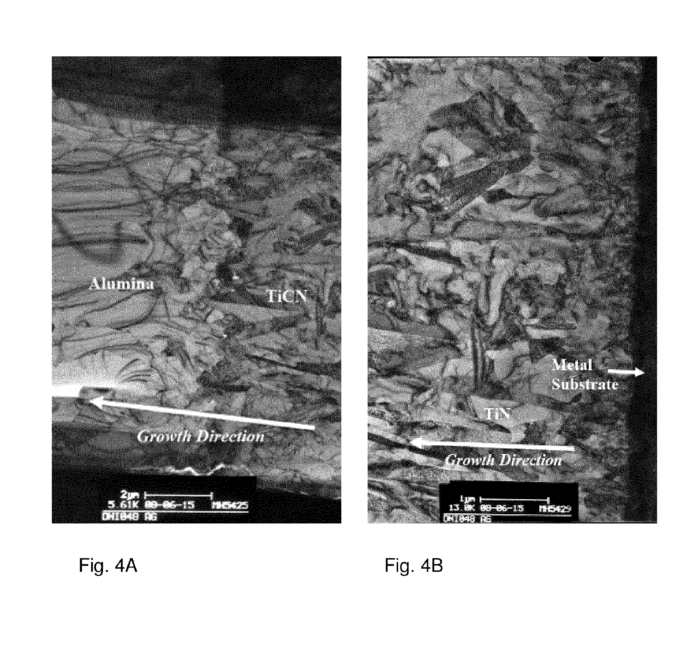

FIGS. 4A and 4B provide photographs acquired by transmission electron microscope (TEM) imaging of a conventional, "dual layer" coating (an inner band comprising a single layer of TiN and a middle band comprising a single layer of TiCN, with an Al₂O₃ overcoat as the outer band) on a metal substrate. In the conventional structure, the TiN layer and the TiCN layer both have a thickness of about 2.5 µm, and the Al₂O₃ overcoat has a thickness of about 5 µm. It was observed that the dual layer structures consist of relatively large, high aspect ratio grains of TiN and TiCN (up to 2 to 3 µm in the growth direction).

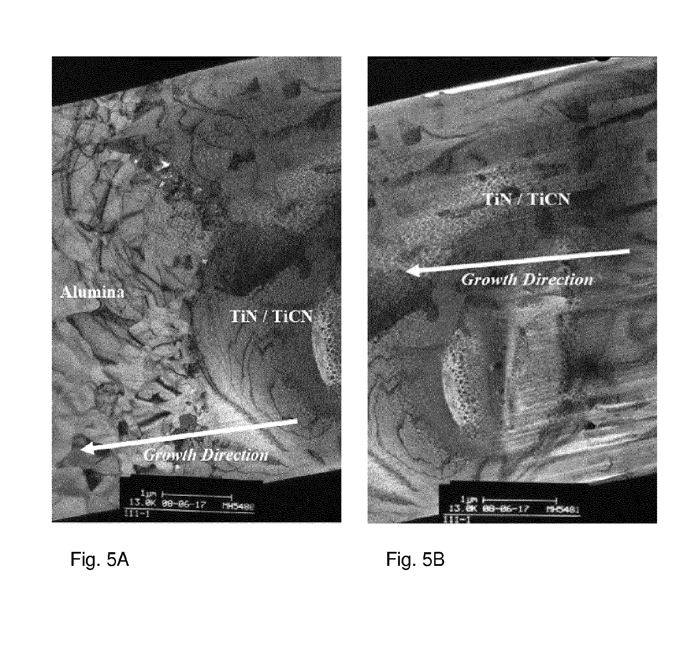

FIGS. 5A and 5B provide TEM images of multilayer TiN/TiCN coatings on a metal substrate in accordance with the invention. The coatings depicted in FIGS. 5A and 5B comprise a first band comprising a single layer of TiN having a thickness of 1 µm, a second band comprising a layer of TiCN (having a thickness of about 0.1 µm) on top of the first TiN band/layer, and, on top of the second layer, alternating subsequent layers of TiN and TiCN, each subsequent layer having a thickness of about 0.1 µm. The total thickness of the middle band in the multilayer structure is about 5 µm. The structure also includes an outer band comprising an Al₂O₃ overcoat having a thickness of about 5 µm (visible in FIG. 5A). In clear contrast with the conventional structure, the images of the multilayer coating revealed that the grains of TiN and TiCN were so small that they could not be distinguished as discrete elements within the TiN/TiCN multilayer structure. Accordingly, observations of improved microstructure and grain morphology were made, with larger acicular grains growing perpendicular to the substrate in the conventional dual layer structure being replaced by very fine, randomly-oriented grains in the present multilayer coating structure. Such features suggest that the multilayer coatings on pressed/ homogenised/ground metal substrates of the present invention may improve fracture toughness and resistance to the growth of microcracks, at least by reducing grain size and changing morphology within the coating film. It appears that smaller, randomly oriented grain structure in the present coatings might provide improved mechanical performance by removing anisotropic nature of the TiN and TiCN in the coating.

As the above tests indicate, aggressively scratched ceramic coatings that include a band of multiple thin layers formed on CoCrMo substrates display consistently greater corrosion and scratch resistance when the CoCrMo substrate has been pressed (using hot isostatic pressing), homogenised, ground (rough and CNC) and polished prior to being coated with the ceramic material. It is anticipated that use of such coatings on orthopaedic implant components will demonstrate improved wear resistance *in vivo* as well. In addition, FIGS. 9A and 9B illustrate that even for more conventional ceramic coated substrates, with 2 layers of TiN and TiCN and an Al₂O₃ overcoat, visible defects can be reduced by pressing, homogenizing, grinding (rough and CNC) and polishing prior to coating the substrate with the ceramic material. It is anticipated that this improvement will be realised when applied to orthopaedic implant components as well.

## Claims

1. A method of making an orthopaedic implant component comprising the steps of:
obtaining a metal orthopaedic implant component that has been pressed using hot isostatic pressing and homogenised,
depositing a ceramic coating on the pressed and homogenised component by:
depositing a first band of the ceramic coating upon the pressed and homogenised metal substrate, and
depositing a second band of the ceramic coating upon the first band of the ceramic coating.

2. The method of claim 1 in which the step of obtaining a metal orthopaedic implant component that has been pressed and homogenised comprises obtaining a metal orthopaedic implant component with a surface that has been pressed, homogenised and from which 0.5 to 1 mm of pressed and homogenised metal has been removed from at least a portion of the metal orthopaedic implant component.

3. The method of claim 2 in which at least a portion of the 0.5 to 1 mm of pressed and homogenised metal has been removed using at least one of grinding, machining and polishing.

4. The method of claim 1 in which the first band comprises a plurality of layers of titanium nitride, titanium carbonitride or both titanium nitride and titanium carbonitride, which preferably have been deposited using a chemical vapour deposition technique.

5. The method of claim 4 in which the second band comprises alumina and in which the alumina defines the outer articular surface of the orthopaedic implant component.

6. The method of claim 5 which includes depositing a bonding band between the first band and the alumina of the second band.

7. The method of claim 4 in which the step of depositing a second band comprises depositing by chemical vapour deposition a plurality of layers, each layer comprising titanium nitride, titanium carbonitride or both titanium nitride and titanium carbonitride.

8. The method of claim 7 in which the plurality of layers includes at least 2 layers, preferably at least 5 layers, more preferably at least 30 layers.

9. The method of claim 7 in which the plurality of layers includes not more than 100 layers, preferably not more than 50 layers.

10. The method of claim 7 which includes depositing a third band of the ceramic coating upon the second band of the ceramic coating.

11. The method of claim 10 in which:
the third band comprises alumina,
the alumina defines the outer articular surface of the orthopaedic implant component, and
the third band is deposited by chemical vapour deposition.

12. The method of claim 11 which includes depositing a bonding band between the second band and the alumina of the third band.
